# DEMANDE DE BREVET EUROPEEN

(11) **EP 0 821 917 A1**
(43) Date de publication de la demande: **04.02.1998**
(21) Numéro de dépôt: 97420134.5
(22) Date de dépôt: 28.07.1997
(51) Int. Cl.: A61B 17/70

(54) **Dispositif pour relier et assister mécaniquement des vértebres entre elles**

(30) Priorité: 01.08.1996 FR 9609913
(71) Demandeur: Graf, Henry, F-69006 Lyon (FR)
(72) Inventeur: Graf, Henry, F-69006 Lyon (FR)
(74) Mandataire: Myon, Gérard Jean-Pierre

(57) **Abrégé**

L'invention concerne un dispositif pour relier et assister mécaniquement des vertèbres entre elles, comprenant au moins un dispositif d'attache (1) comportant une rotule (20) solidaire d'une vertèbre et un corps compressible (9) maintenu suivant l'axe longitudinal dudit dispositif d'attache, ladite rotule et ledit élément compressible étant logés entre deux corps d'extrémité (3, 18) dudit dispositif d'attache, caractérisé en ce que l'un au moins desdits corps d'extrémité est formé par une cupule (3, 18) apte à coiffer ladite rotule (20) et en ce que lesdits corps d'extrémité sont reliés entre eux par un élément de liaison (8') disposé autour dudit corps compressible et sur au moins une desdites cupules, ledit élément de liaison étant apte à se déplacer sur la face externe de ladite cupule.

## Description

La présente invention concerne un dispositif pour relier et assister mécaniquement des vertèbres entre elles, comprenant au moins un dispositif d'attache comportant une rotule solidaire d'une vertèbre et un corps compressible maintenu suivant l'axe longitudinal dudit dispositif d'attache, ladite rotule et ledit élément compressible étant logés entre entre deux corps d'extrémité dudit dispositif d'attache.

Ce type de dispositif pour relier et assister mécaniquement des vertèbres entre elles agit en dehors des disques vertébraux afin de compenser des défauts du rachis, ce qui permet le contrôle et/ou la modification des mouvements de flexion et d'extension entre deux vertèbres.

On connaît, par le EP-A-0 611 554, un amortisseur intervertébral constitué par une chambre cylindrique recevant une rotule solidaire d'une vertèbre, un piston susceptible de coulisser dans cette chambre cylindrique ainsi qu'un corps compressible. Cette chambre cylindrique est fermée, à son extrémité opposée au corps compressible, par une partie discoïdale vissée. Le volume du corps élastique, au repos, est inférieur à celui du volume déterminé par la position du piston par rapport au fond de la chambre cylindrique.

Ce dispositif permet une bonne stabilisation intervertébrale grâce à la présence du corps compressible qui résiste progressivement de manière exponentielle à l'avance du piston. La liaison entre deux dispositifs de ce type est réalisée par une bague d'assemblage coopérant avec des tenons réalisés sur les extrémités correspondantes des chambres cylindriques.

Pour permettre un débattement relatif en rotation, entre deux dispositifs d'attache voisins, selon les trois dimensions de l'espace, il est nécessaire que cette bague d'assemblage soit réalisée en un matériau permettant des torsions suivant ces trois directions. Cette organe d'assemblage doit donc présenter une rigidité suffisante pour relier deux dispositifs d'attache voisins, ainsi qu'une souplesse importante lui permettant de se tordre suivant trois directions. Ce compromis rend la conception d'une telle bague très délicate, et son prix de revient élevé.

Afin de pallier ces différents inconvénients, la présente invention vise la réalisation d'un dispositif pour relier et assister mécaniquement des vertèbres entre elles, dans lequel les dispositifs d'attache peuvent se déplacer mutuellement en rotation selon les trois directions de l'espace, et qui présente une structure simple et fiable.

A cet effet, l'invention a pour objet un dispositif du type précité, caractérisé en ce que l'un au moins des corps d'extrémité est formé par une cupule apte à coiffer la rotule, et en ce que les corps d'extrémité sont reliés entre eux par un élément de liaison disposé autour du corps compressible et sur au moins une des cupules, l'élément de liaison étant apte à se déplacer sur la face externe de la cupule.

On entend par cupule un organe dont la face intérieure est sensiblement sphérique pour coopérer avec la rotule, et dont la face extérieure est elle aussi sensiblement sphérique pour coopérer avec l'élément de liaison.

Le dispositif conforme à l'invention permet de réaliser les objectifs précédemment mentionnés. En effet, puisque l'élément de liaison peut se déplacer sur la face externe de la cupule, les deux corps d'extrémité de chaque dispositif d'attache sont maintenus selon une direction axiale mais peuvent se déplacer mutuellement en rotation selon les trois direction de l'espace. De la sorte, il n'est pas nécessaire de faire appel à un organe supplémentaire permettant le débattement entre les extrémités adjacentes de deux dispositifs d'attache voisins. Ces extrémités adjacentes peuvent par exemple être laissées solidaires. Etant donné qu'un organe supplémentaire n'est plus obligatoire, chaque dispositif d'attache présente alors des dimensions longitudinales plus importantes, ce qui en rend la conception plus aisée, la solidité accrue et l'efficacité améliorée. En particulier, le corps compressible peut être de dimensions plus importantes que dans l'art antérieur.

Selon une première variante de l'invention, l'élément de liaison de chaque dispositif d'attache comporte une embase à partir de laquelle s'étendent trois branches présentant, à leurs extrémités libres, une surface de contact avec la cupule, un anneau réunissant les branches entre elles, tandis que l'embase est pourvue d'un logement coopérant avec un doigt de la chambre de compression pour constituer un dispositif de verrouillage à baïonnette.

Selon une autre variante de l'invention, l'élément de liaison de chaque dispositif d'attache, comporte un manchon dont l'une des extrémités présente une fente coopérant avec un doigt solidaire avec la chambre de compression pour constituer un dispositif de verrouillage à baïonnette, tandis qu'à l'opposé de la fente, le manchon présente une ouverture oblongue pour le passage de la rotule de la vis correspondante.

Ces modes de réalisation faisant intervenir un verrouillage à baïonnette permettent le maintien de la chambre de compression selon son axe longitudinal, ainsi qu'une rotation de l'élément de liaison par rapport à cette chambre, autour de cette axe longitudinal.

Dans le cas où un dispositif de verrouillage à baïonnette n'est pas utilisé, l'élément de liaison de chaque dispositif d'attache, peut être constitué de deux éléments réunis l'un à l'autre autour de la chambre de compression de la cupule, par l'intermédiaire de pions de retenue.

Ce mode de réalisation présente une grande simplicité de fabrication, et assure une liaison aisément amovible.

Dans les cas où l'un des corps d'extrémité est formé par une calotte, les surfaces de contact correspondantes de la calotte et de l'élément de liaison sont de préférence sensiblement sphériques, ce qui assure un auto-réglage de la chambre de compression suivant son axe longitudinal.

Selon un autre mode de réalisation de l'invention, les deux corps d'extrémité sont des cupules et l'élément de liaison comprend deux anneaux ouverts élastiques reliés l'un à l'autre par des travers, chaque anneau étant disposé sur la face externe d'une cupule correspondante. Ce mode de réalisation présente une grande simplicité, dans la mesure où les anneaux élastiques peuvent, par simple clipsage, à la fois être maintenus sur la face externe des cupules et en être otés.

Selon une caractéristique avantageuse de l'invention, les extrémités adjacentes de deux dispositifs d'attache voisins sont réalisées d'un seul tenant, ce qui assure une simplicité de fabrication appréciable. Dans ce cas, chaque extrémité d'un dispositif d'attache est susceptible de se déplacer en rotation suivant les trois directions d'espace, par rapport à l'extrémité distale du dispositif d'attache voisins, ce qui assure une parfaite possibilité de mouvement relatif entre ces deux dispositifs d'attache voisins.

Le dessin annexé, donné à titre d'exemple, permettra de mieux comprendre l'invention, les caractéristiques qu'elle présente et les avantages qu'elle est susceptible de procurer:
- La figure 1 est une vue montrant deux dispositifs d'attache constituant le dispositif pour relier et assister des vertèbres entre elles.
- La figure 1a est une vue illustrant une variante de l'élément de liaison de chaque dispositif d'attache suivant l'invention.
- La figure 2 est une vue en perspective éclatée montrant une variante d'un dispositif d'attache ;
- La figure 3 est une vue représentant le dispositif d'attache de figure 2 en position montée ;
- La figure 4 est une vue semblable à celle de figure 2, mais représentant une seconde variante du dispositif d'attache ;
- Les figures 5 et 6 sont des vues montrant le dispositif d'attache représenté en figure 4 en position montée;
- Les figures 7 à 9 sont des vues illustrant une troisième variante du dispositif d'attache de figure 1 ;
- La figure 10 est une vue en perspective partielle, illustrant une variante de réalisation et de montage du dispositif d'attache et
- La figure 11 est une coupe axiale de la rotule représentée à la figure 10.

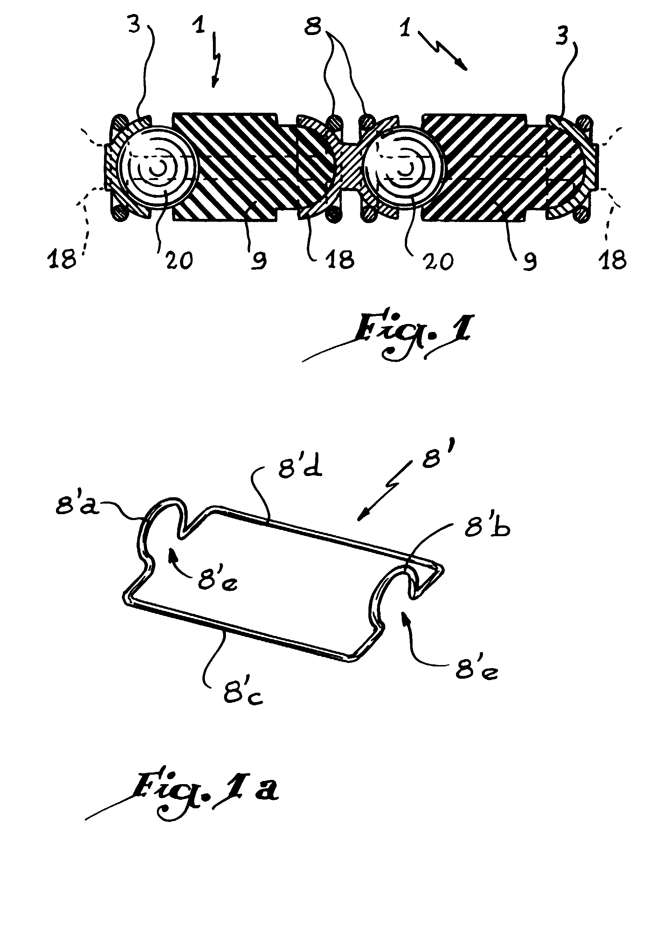

La figure 1 représente un dispositif pour relier et assister mécaniquement des vertèbres entre elles conforme à l'invention. Il est constitué de deux dispositifs d'attache désignés dans leur ensemble par les référence 1 et 1'.

Chaque dispositif d'attache comprend une rotule 20 solidaire d'une vis intervertébrale non représentée qui est implantée au préalable dans une vertèbre. Une cupule 3, formant un premier corps d'extrémité, coiffe un premier côté de la rotule 20. Un corps compressible 9 est disposé sur le côté de la rotule opposé à la cupule 3. Ce corps compressible est un corps élastique susceptible de modifier sa largeur sur l'action d'un effort de compression. Il coopère avec la rotule 20 par l'intermédiaire d'une cavité sphérique correspondante. Le corps compressible est en outre coiffé, à son extrémité opposée à la rotule, par une cupule 18 formant un second corps d'extrémité du dispositif d'attache 1.

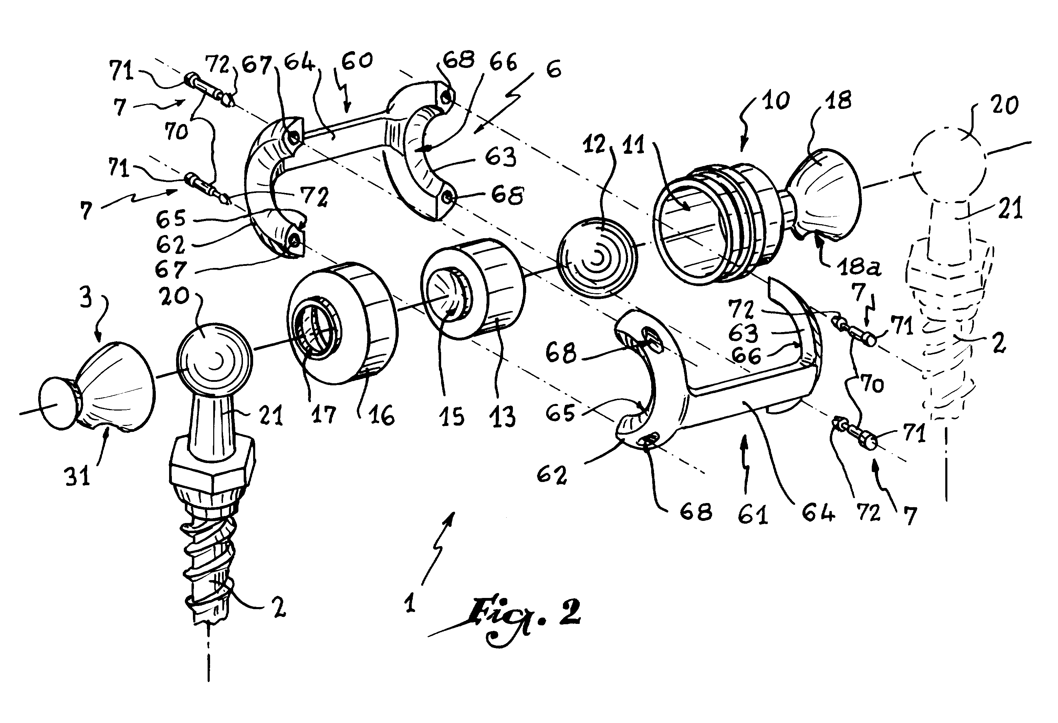

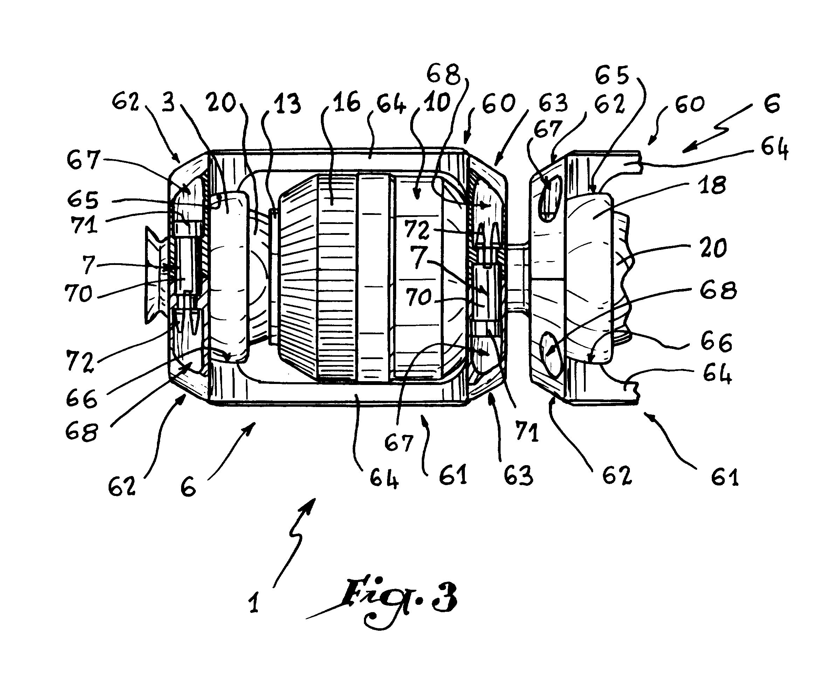

Les cupules d'extrémité 3, 18 sont reliées entre elles par l'intermédiaire d'un élément de liaison 6 conforme à celui décrit aux figures 2 et 3. Cet élément de liaison 6 est susceptible de se déplacer sur les faces externes des cupules 3, 18 de sorte que ces dernières, tout en étant reliées suivant une direction sensiblement axiale, sont susceptibles de se déplacer mutuellement selon des rotations suivant les trois directions de l'espace.

La cupule 18 se prolonge, à l'opposé de la rotule 20, par une cupule 3' analogue à celle 3, qui constitue un premier corps d'extrémité du second dispositif d'attache 1', ce dernier étant analogue au dispositif 1 décrit ci-dessus.

Conformément à l'invention, les deux dispositifs d'attache 1, 1' sont susceptibles de se déplacer mutuellement en rotation suivant les trois directions de l'espace.

L'élément de liaison 6 de chaque dispositif d'attache 1 peut être remplacé par celui 8' représenté à la figure 1a. Ce dernier comprend deux anneaux ouverts et opposés 8'a et 8'b reliés entre eux par des traverses parallèles 8'c et 8'd. Les anneaux 8'a et 8'b présentent chacun une ouverture 8'e de dimensions inférieures à celles de la partie rétreinte 33, 18c des cupules 3 et 18 pour permettre une fixation par déformation élastique de l'élément de liaison 8'.

On a représenté en figures 2 et 3 une variante de réalisation dans laquelle chaque dispositif d'attache 1 comprend une cupule 3 formant un premier corps d'extrémité, qui coiffe une rotule 20 d'une vis intervertébrale ou crochet 2 préalablement implanté dans une vertèbre. Une calotte 11 formant un second corps d'extrémité, coopère avec un soufflet 16 pour former une chambre de compression 10. Cette dernière reçoit un corps élastique ou compressible 12 dont le volume est inférieur à celui de la calotte 11 de la chambre 10. Le corps élastique 12 affecte une forme sphérique ou analogue déformable lors de mouvements en flexion/extension entre deux vertèbres d'une colonne vertébrale. La combinaison d'au moins deux dispositifs d'attache 1 permet un meilleur contrôle des efforts appliqués longitudinalement selon l'axe du corps compressible. En effet les dispositifs d'attache 1 suivant l'invention permettent la transformation en compression de tout effort appliqué longitudinalement à l'axe du corps compressible, que l'effort soit une distraction et/ou une compression.

Dans la chambre de compression 10 coulisse un piston 13 présentant une première cavité en portion de sphère 14 dont le rayon est supérieur à celui du corps élastique 12 en position de repos. A l'opposé de la cavité 14, le piston 13 comporte une autre cavité 15 en portion de sphère qui coopère avec la rotule 20. Cette vis peut également constituer l'extrémité d'un système rigide visant à solidariser plusieurs vertèbres entre elles.

Le soufflet 16 présente une ouverture 17 qui entoure de manière étanche la cavité 15 du piston 13 pour que ce dernier soit en contact direct avec la rotule 20.

A l'opposé du piston 13 est prévue une cupule 3 qui enveloppe partiellement la rotule 20 de la vis 2.

On note que la calotte 11 se prolonge à l'opposé du corps 12 par une cupule 18 d'un second dispositif d'attache identique à celle 3, pour venir coopérer avec la rotule 20 d'une vis 2. La liaison entre deux dispositifs d'attache s'effectue donc d'un seul tenant.

Les cupules 3 et 18 présentent une ouverture 31 et 18a permettant le passage de la tige 21 de la vis 2 supportant la rotule 20. Les cupules 3 et 18 présentent une partie interne 32 et 18b en portion de sphère de rayon identique à celui de la rotule 20 de chaque vis 2.

Un élément de liaison 6 est prévu pour relier la chambre de compression 10 à la cupule 3.

L'élément de liaison 6 est constitué de deux éléments 60 et 61 qui sont réunis l'un à l'autre par l'intermédiaire de pions 7. Les éléments 60 et 61 comportent à chaque extrémité deux demi-couronnes 62 et 63 qui sont réunies l'une à l'autre par l'intermédiaire d'une branche horizontale 64.

Les demi-couronnes 62 et 63 comportent respectivement un profil courbe 65 et 66 qui vient en contact avec la cupule 3 ou 18 et la chambre de compression 10 lors de la fixation des deux éléments 60 et 61.

On constate que lors de l'assemblage des deux éléments 60 et 61, les demi-couronnes 62, 63 de l'élément 60 sont en contact respectivement avec celles 62, 63 de l'élément 61 pour que les profils courbes 65 et 66 se prolongent sur tout le pourtour des cupules 3 ou 18 et de la chambre de compression 10. Ainsi, la demi-couronne 62 de l'élément 60 est en contact avec la demi-couronne 62 de l'élément 61 et de même pour les demi-couronnes 63.

Les demi-couronnes 62 et 63 de l'élément 60 sont percées respectivement de trous débouchants 67 et 68, tandis que les demi-couronnes 62 et 63 de l'élément 61 sont respectivement percées de trous 68 et 67.

Les trous 67 et 68 permettent le passage du pion de retenue 7 pour l'assemblage des éléments 60 et 61 de l'élément 6. Les pions 7 comportent un corps allongé 70 solidaire d'une tête 71 et d'une pointe fendue 72 permettant sa retenue dans le trou 67, 68 correspondant.

On constate que la cupule 18 solidaire de la chambre de compression 10 coopère d'une part avec la rotule 20 d'une vis 2 et d'autre part avec un élément de liaison 6 du second dispositif d'attache 1 pour constituer le dispositif suivant l'invention (figure 9).

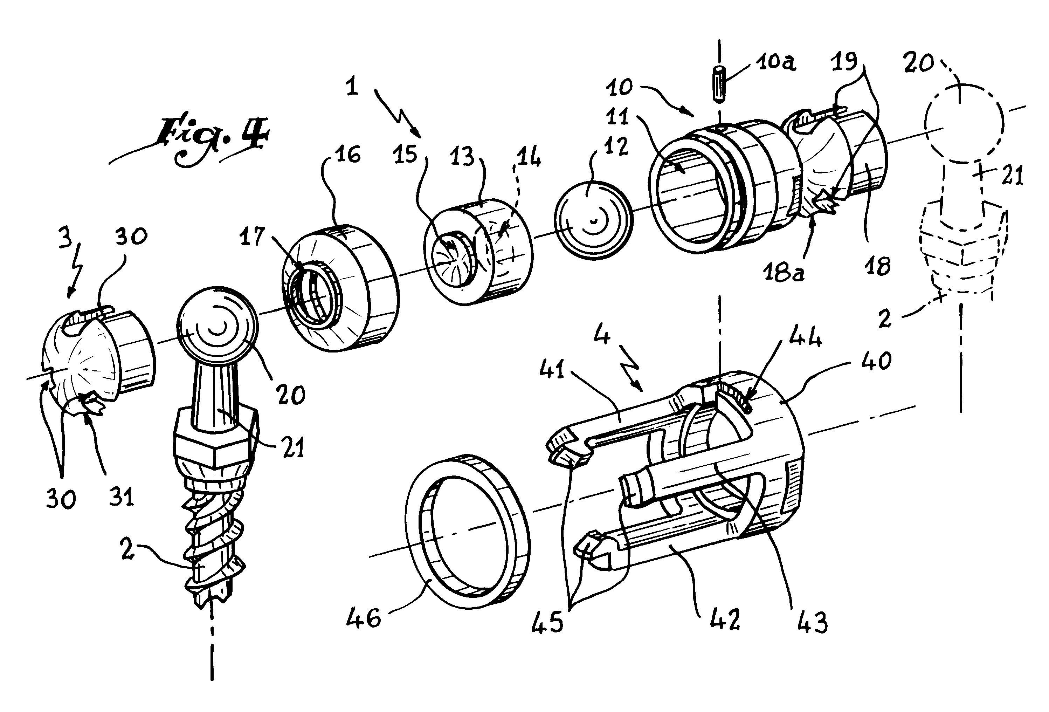

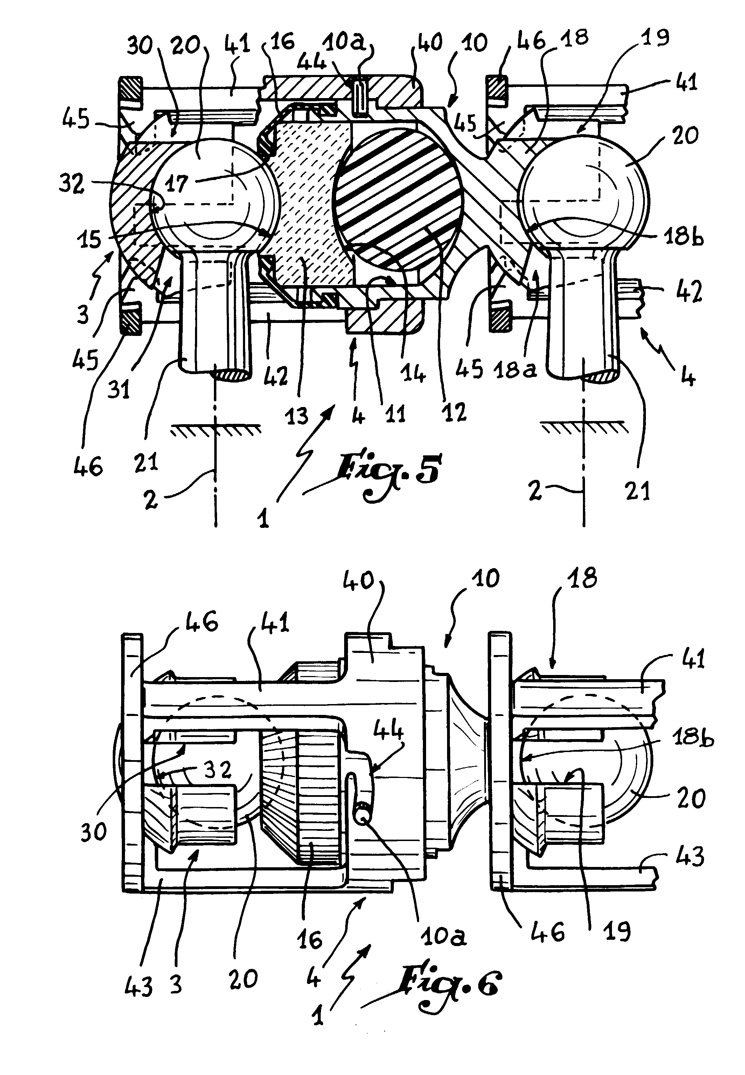

On a représenté en figures 4 à 6 une seconde variante dans laquelle chaque dispositif d'attache 1 présente un élément de liaison ou cage 4 de configuration différente de celui 6 décrit préalablement, tandis que tous les autres éléments permettant la retenue du corps élastique 12 dans la chambre de compression 10 sont identiques ou similiares.

La cage 4 comprend une embase ou base cylindrique 40 solidaire de trois branches 41 à 43 qui s'étendent longitudinalement et parallèlement à l'axe du dispositif 1. La base 40 présente un logement 44 dans lequel pénètre au moins un doigt 10a solidaire de la chambre de compression 10 pour constituer un dispositif de verrouillage à baïonnette lorsque la cage 4 est entraînée en rotation autour de ladite chambre.

A chaque extrémité libre des branches 41 à 43 est prévue une surface de contact ou de butée 45 qui vient en appui contre la face externe de la cupule 3 par l'intermédiaire d'un anneau de pression 46.

En effet, lors du montage du dispositif d'attache 1, on remarque que les branches 41 à 43 coopèrent, avant rotation, avec les échancrures 30 de la cupule 3 pour que la butée 45 de chaque branche 41 à 43 vienne lors de la rotation de la cage 4 en contact avec la face externe de ladite cupule. Lors de cette rotation, le doigt 10a de la chambre 10 coopère simultanément avec le logement 44 de la cage 4.

On remarque que les cupules 3 et 18 présentent respectivement sur leur pourtour des échancrures 30 et 19 espacées à intervalles réguliers.

On remarque que la cupule 18 solidaire de la chambre de compression 10 coopère d'une part avec la rotule 20 d'une vis 2 et d'autre part avec une cage 4 du second dispositif d'attache 1 afin de constituer le dispositif suivant l'invention.

On constate ainsi qu'entre deux rotules 20 de deux vis 2 préalablement implantées dans deux vertèbres voisines, les dispositifs d'attache 1 assemblés les uns aux autres peuvent pivoter librement autour de chaque rotule 20 correspondante.

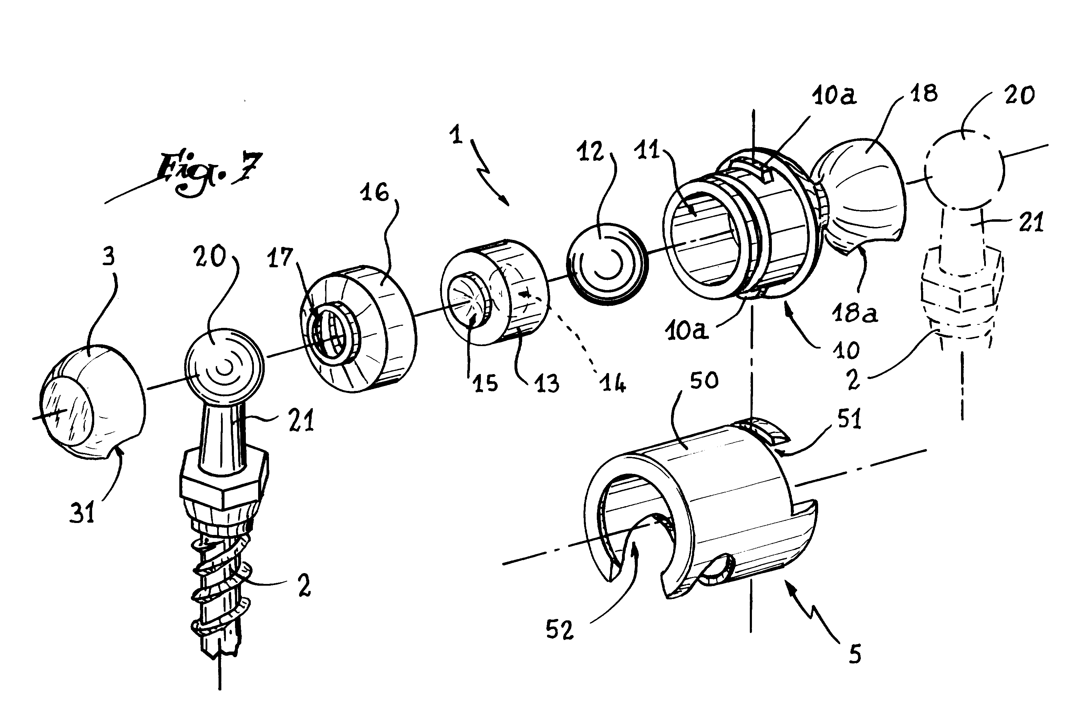

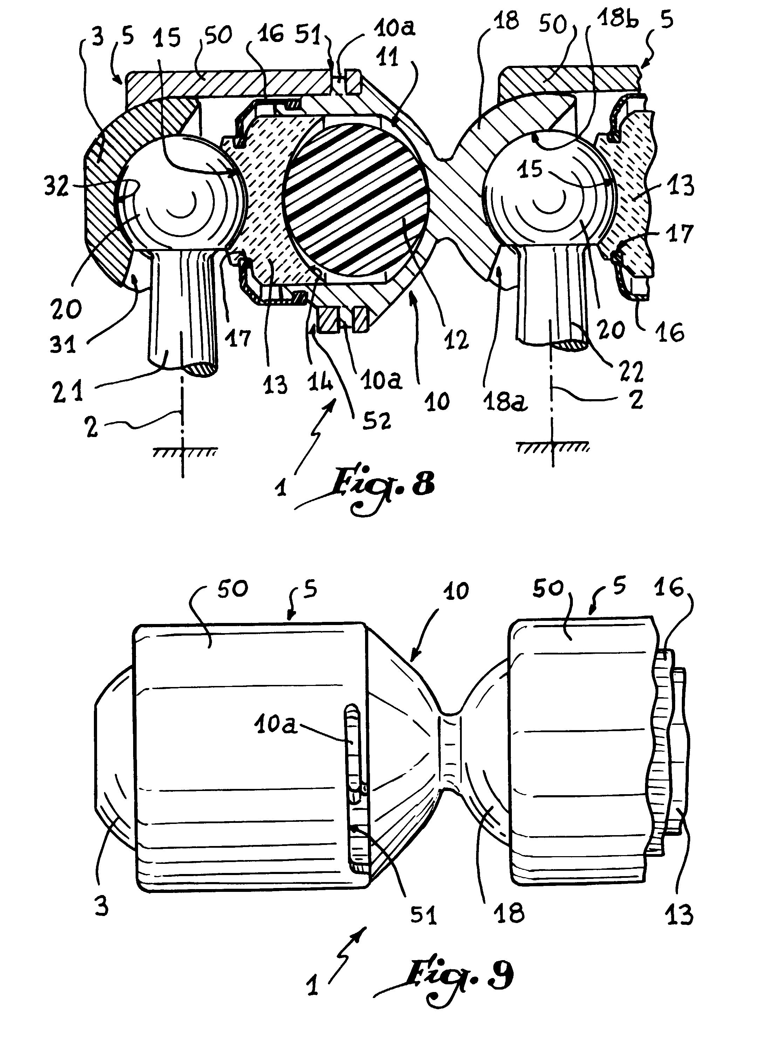

On a représenté en figures 7 à 9 une troisième variante du dispositif suivant l'invention.

Chaque dispositif d'attache 1 présente un élément de liaison ou cage 5 de configuration différente de celle 4 décrite préalablement, tandis que tous les autres éléments permettant la mise en place et la retenue du corps élastique 12 sont identiques ou similaires.

En effet, le corps élastique 12 est maintenu dans la partie interne 11 de la chambre de compression 10 par l'intermédiaire du piston 13 et du soufflet d'étanchéité 16. La rotule 20 de la vis 2 correspondante est retenue entre la cavité 15 du piston 13 et la cupule 3 par l'intermédiaire de la cage 5.

La cage 5 est constituée d'un manchon cylindrique 50 ouvert à chacune de ses extrémités. Le manchon 50 présente à l'une de ses extrémités une fente 51, tandis que l'extrémité opposée comporte une ouverture oblongue 52 pour le passage de la tige 21 supportant la rotule 20 de la vis 2 correspondante.

Ainsi la chambre de compression 10 munie du corps élastique 12, du piston 13 et du soufflet d'étanchéité 16 sont introduits à l'intérieur de la cage 5 du côté de l'ouverture comportant l'extrémité oblongue 52 de manière que la cupule 18 débouche à l'extérieur du manchon 50.

Ensuite, la rotule 20 de la vis 2 et la cupule 3 sont placées à l'intérieur de la cage 5 de manière que ladite rotule soit en appui contre la cavité 15 du piston 13. L'ensemble est maintenu dans la cage 5 par rotation de cette dernière autour de son axe longitudinal pour engager le doigt 10a solidaire de la chambre de compression 10 à l'intérieur de la fente 51 afin de constituer un dispositif de verrouillage à baïonnette.

Cette variante du dispositif suivant l'invention constitué d'au moins deux dispositifs d'attache 1 peut pivoter librement autour de la rotule 20 de la vis 2 correspondante.

On note que les cupules 3 et 18 ne comportent pas d'échancrure 30 et 19 comme cela est décrit précédemment en figures 4 à 6, du fait de la configuration de la cage 5.

On constate que la cupule 18 coopère, comme déjà décrit préalablement, d'une part avec la rotule 20 d'une vis 2 et d'autre part avec une cage 5 du second dispositif d'attache 1 pour constituer le dispositif suivant l'invention.

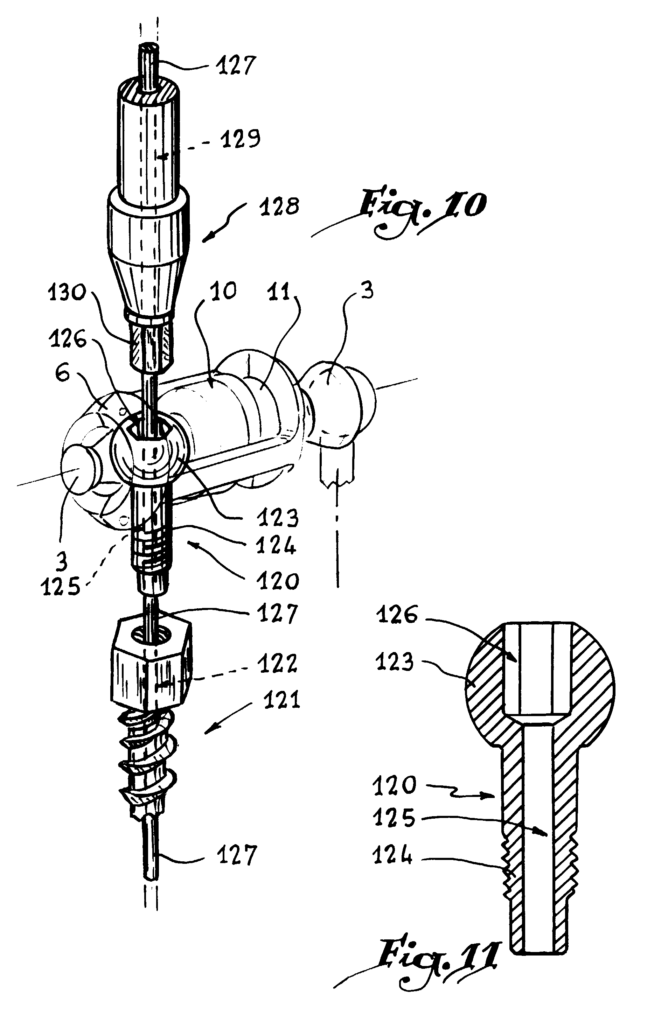

La figure 10 montre une vue en perspective partielle d'un dispositif d'attache conforme à l'invention en cours de pose. Il comprend un ensemble dont les extrémités formées d'une cupule 3 et d'une calotte 11 sont reliées par un élément de liaison 6 conforme à celui décrit aux figures 2 et 3.

Une rotule 120 du dispositif d'attache 1 est prévue pour être vissée sur une tige 121 solidaire d'une vertèbre non représentée.

Cette rotule 120 et cette tige 121 comprennent des moyens permettant leur fixation mutuelle par le chirurgien, durant l'opération. A cet effet, la tige 121 est percée d'un orifice axial 122 s'étendant sur une partie de sa hauteur.

La rotule 120 comprend, comme le montre plus clairement la figure 11, une tête sphérique 123 et une queue 124 cylindrique filetée, elle aussi percée d'un orifice axial 125 traversant, de diamètre voisin de celui de l'orifice 122.

La tête 123 de la rotule est pourvue d'une cavité 126 en relation avec l'orifice 125, dont la section transversale est polygonale, par exemple hexagonale.

Le montage du dispositif d'attache 1 sur la tige 121 implantée au préalable dans une vertèbre, est le suivant.

On introduit tout d'abord une broche guide 127 dans l'orifice 122 de la tige 121. On fait ensuite descendre le dispositif d'attache assemblé au préalable, grâce à cette broche qui pénètre par l'orifice 125 et la cavité 126 de la rotule 120, jusqu'à ce que les extrémités libres de la queue 124 et de la tige 121 soient en contact.

On fait alors descendre un outil 128 pourvu d'un orifice axial 129 traversant, le long de la broche guide 127. Cet outil présente une tête 130 de forme complémentaire de la cavité 126. On tourne enfin l'outil 128 ce qui, par coopération de la tête 130 et de la cavité 126, permet de visser la queue de la rotule sur la tige. Ce vissage n'induit aucun déplacement de l'ensemble du dispositif d'attache, puisque la tête de la rotule est libre de tourner autour de l'axe de sa queue, par rapport aux autres éléments du dispositif d'attache.

L'outil et la broche guide sont alors retirés.

On peut, de manière analogue, monter plusieurs dispositifs d'attache assemblés au préalable entre eux, en utilisant un nombre correspondant de broches guides.

Le montage serait analogue dans le cas où la rotule serait constituée uniquement par sa tête 123 et dépourvue de queue.

L'homme du métier devrait alors prévoir une cavité polygonale de plus faible dimension axiale, de manière à réaliser un taraudage de réception de la tige, à l'extrémité inférieure de la tête de la rotule.

On peut prévoir que le corps compressible 9 soit remplacé par un corps incompressible suivant les cas pathologiques.

Le dispositif pour relier et assister mécaniquement des vertèbres entre elles constitué d'au moins deux dispositifs d'attache 1 décrits précédemment joue le rôle d'une prothèse d'assistance discale en position extradiscale.

Le dispositif suivant l'invention contrôle les variations de distances relatives en éloignement et/ou rapprochement entre deux points fixes (rotules implantées chacune dans deux vertèbres voisines et adjacentes).

On entend par libre déplacement de chaque dispositif d'attache 1 d'une part une rotation autour de chaque rotule 20, mais également une rotation de chaque élément de liaison 8', 4, 5, 6 autour des cupules 3, 18 et d'autre part des mouvements de translation suivant l'axe longitudinal du dispositif pouvant aboutir à des désolidarisations transitoires des éléments entre eux lors de certains efforts.

La combinaison des rotations et des désolidarisations peut aboutir à des mouvements complexes.

## Revendications

1. Dispositif pour relier et assister mécaniquement des vertèbres entre elles, comprenant au moins un dispositif d'attache (1) comportant une rotule (20 ; 120) solidaire d'une vertèbre et un corps compressible (9) maintenu suivant l'axe longitudinal dudit dispositif d'attache, ladite rotule et ledit élément compressible étant logés entre deux corps d'extrémité (3, 18 ; 3, 11) dudit dispositif d'attache, caractérisé en ce que l'un au moins desdits corps d'extrémité est formé par une cupule (3, 18) apte à coiffer ladite rotule et en ce que lesdits corps d'extrémité sont reliés entre eux par un élément de liaison (4 ; 5 ; 6 ; 8') disposé autour dudit corps compressible et sur au moins une desdites cupules, ledit élément de liaison étant apte à se déplacer sur la face externe de ladite cupule.

2. Dispositif pour relier et assister mécaniquement des vertèbres entre elles, comprenant au moins deux dispositifs d'attache (1) réunis l'un à l'autre, caractérisé en ce que chaque dispositif d'attache comporte des cupules (3, 18) aptes à coiffer une rotule (20 ; 120) solidaire d'une vertèbre et reliées entre elles par l'intermédiaire d'un élément de liaison (4 ; 5 ; 6 ; 8'), tandis qu'un corps compressible (9) est maintenu suivant l'axe longitudinal de l'élément de liaison entre lesdites cupules (3, 18), ledit élément de liaison étant disposé autour dudit corps compressible et d'une au moins desdites cupules, ledit élément de liaison étant libre de se déplacer sur la face externe de ladite cupule.

3. Dispositif suivant la revendication 1 ou 2, caractérisé en ce qu'un seul des corps d'extrémité est une cupule (3), l'autre corps d'extrémité étant une calotte (11) destinée à coopérer avec un soufflet (16) pour former une chambre de compression (10) dans laquelle est disposée le corps compressible.

4. Dispositif suivant la revendication 3, caractérisé en ce que la chambre de compression (10) reçoit en outre un piston (13) disposé contre le soufflet (16), ce dernier présentant un orifice (17) de sorte que ledit piston est en contact direct avec la rotule (20) correspondante.

5. Dispositif suivant la revendication 3 ou 4, caractérisé en ce que l'élément de liaison (4) de chaque dispositif d'attache (1) comporte une embase (40) à partir de laquelle s'étendent trois branches (41 à 43) présentant à leurs extrémités libres, une surface de contact (45) avec ladite cupule, un anneau réunisant les branches (41 à 43) entre elles, tandis que la base (40) présente un logement (44) coopèrant avec un doigt (10a) de la chambre de compression (10) pour constituer un dispositif de verrouillage à baïonnette.

6. Dispositif suivant la revendication 3 ou 4, caractérisé en ce que l'élément de liaison (5) de chaque dispositif d'attache (1) comporte un manchon (50) dont l'une des extrémités présente une fente (51) coopérant avec un doigt (10a) solidaire de la chambre de compression (10) pour constituer un dispositif de verrouillage à baïonnette, tandis qu'à l'opposé de la fente (51), le manchon (50) présente une ouverture oblongue (52) pour le passage de la rotule (20) correspondante.

7. Dispositif suivant la revendication 3 ou 4, caractérisé en ce que l'élément de liaison (6) de chaque dispositif d'attache (1) est constitué de deux éléments (60, 61) qui sont réunis l'un à l'autre autour de la chambre de compression (10) et de la cupule (3, 18) par l'intermédiaire de pions de retenue (7).

8. Dispositif suivant la revendication 8, caractérisé en ce que chaque élément (60 et 61) est constitué de deux demi-couronnes (62, 63) reliées l'une à l'autre par une branche horizontale (64).

9. Dispositif selon l'une quelconque des revendications 3 à 8, caractérisé en ce que les surfaces de contact mutuelles de la calotte (11) et de l'élément de liaison (4 ; 5 ; 6) sont sensiblement sphériques.

10. Dispositif selon la revendication 1 ou 2, caractérisé en ce que les deux corps d'extrémité sont des cupules (3, 18) et l'élément de liaison (8') comprend deux anneaux ouverts (8'a, 8'b) élastiques reliés l'un à l'autre pour des traverses (8'c, 8'd), chaque anneau étant disposé sur la face externe de la cupule correspondante.

11. Dispositif selon l'une quelconque des revendications précédentes, caractérisé en ce que les extrémités adjacentes (3, 18 ; 3, 11) de deux dispositifs d'attache sont réalisées d'un seul tenant.

12. Dispositif suivant l'une quelconque des revendications précédentes, caractérisé en ce que la rotule (120) est pourvue d'un orifice traversant (125) de réception d'une broche guide (127), en vue de son montage sur une tige (121) solidaire d'une vertèbre.

13. Dispositif suivant la revendication 12, caractérisé en ce que la rotule (120) est pourvue d'une cavité (126) de manoeuvre, de section transversale polygonale, destinée à coopérer avec un outil (130) de forme complémentaire, en vue de sa fixation sur ladite tige (121).
